(19) Europäisches Patentamt
European Patent Office
Office européen des brevets

(11) EP 0 848 941 B1

(12) **FASCICULE DE BREVET EUROPEEN**

(45) Date de publication et mention de la délivrance du brevet:
**05.07.2006 Bulletin 2006/27**

(51) Int Cl.:
***A61K 8/899*** *(2006.01)* ***A61Q 5/06*** *(2006.01)*

(21) Numéro de dépôt: **97402640.3**

(22) Date de dépôt: **05.11.1997**

(54) **Dispositif aérosol à base de compositions alcooliques de matériaux fixants comprenant des polymères siliconés greffés anioniques**

Aerosolvorrichtung auf der Basis alkoholischer Zusammensetzungen von festigenden Materialien, die anionische gepfropfte Silikonpolymere enthalten

Aerosol device based on alcoholic compositions of binding materials containing anionic grafted silicon polymers

(84) Etats contractants désignés:
**AT BE CH DE DK ES FI FR GB GR IE IT LI LU MC NL PT SE**

(30) Priorité: **22.11.1996 FR 9614331**

(43) Date de publication de la demande:
**24.06.1998 Bulletin 1998/26**

(73) Titulaire: **L'ORÉAL**
**75008 Paris (FR)**

(72) Inventeurs:
- **Samain, Henri**
**91570 Bievres (FR)**
- **Dupuis, Christine**
**75018 Paris (FR)**

(74) Mandataire: **Andral, Christophe André Louis**
**L'OREAL - D.I.P.I.**
**25-29 Quai Aulagnier**
**92600 Asnières (FR)**

(56) Documents cités:
**WO-A-95/03776**
**WO-A-96/21417**
**WO-A-96/32918**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 848 941 B1

## Description

La présente invention concerne de nouveaux dispositifs aérosols destinés à fixer les chevelures.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse, et d'un ou plusieurs matériaux, généralement des résines polymères, dont la fonction est de former des soudures entre les cheveux, appelés encore matériaux fixants, en mélange avec divers adjuvants cosmétiques. Cette solution est généralement conditionnée soit dans un récipient aérosol approprié mis sous pression à l'aide d'un propulseur, soit dans un flacon pompe.

On connaît de nombreux systèmes aérosols destinés à fixer les chevelures, ces systèmes contenant d'une part une phase liquide (ou jus) et d'autre part un propulseur. Le jus contient les matériaux fixants et un solvant approprié. Le propulseur a pour fonction d'assurer une pression permettant la pulvérisation de la phase liquide, et son application sur les cheveux sous forme d'un nuage de gouttelettes dispersées. C'est une fois appliquée sur les cheveux que la phase liquide sèche, permettant la formation de soudures nécessaires à la fixation de la chevelure par les matériaux fixants.

Les soudures doivent être suffisamment rigides pour assurer le maintien des cheveux. Cependant, elles doivent également être suffisamment fragiles pour que l'utilisateur puisse, en peignant ou brossant la chevelure, les détruire sans heurter le cuir chevelu ni endommager les cheveux.

Les matériaux fixants sont généralement des polymères fixants, c'est à dire des polymères filmogènes solubles ou dispersibles dans l'eau et/ou dans l'alcool, tels que les copolymères d'acétate de vinyle/acide crotonique, les résines acryliques anioniques ou amphotères. Ces matériaux permettent d'obtenir facilement l'effet fixant, en revanche, après brossage ou peignage, les cheveux présentent dans les conditions usuelles de laquage un aspect raidi et un toucher rêche, voire collant et un démélage souvent difficile.

Ces inconvénients sont liés à plusieurs paramètres, parmi lesquels on peut citer la nature du ou des polymères fixants, ou encore à la nature des soudures. Pour remédier à ces inconvénients on peut donc agir sur ces deux paramètres, sans toutefois diminuer l'effet fixant recherché. Pour améliorer les propriétés cosmétiques des matériaux fixants, il a surtout été proposé d'associer différents polymères (WO 94/12148, WO 96/06592, US 5 158 762).

La Demanderesse a maintenant trouvé qu'en sélectionnant de manière appropriée d'une part les polymères fixants et d'autre part les paramètres de diffusion des compositions, il était possible d'agir sur la qualité des soudures tout en conservant de bonnes qualités de fixation et/ou de mise en forme la coiffure, et donc apporter d'excellentes propriétés cosmétiques telles que la douceur, le démélage et le toucher.

Le dispositif aérosol selon l'invention est constitué par un récipient contenant une composition aérosol constituée d'une part par une phase liquide (ou jus) contenant au moins un matériau fixant dans un solvant approprié et d'autre part un propulseur, et un moyen de distribution de ladite composition aérosol, le matériau fixant ayant une température de transition vitreuse (Tg) supérieure ou égale à 30 °C, ledit matériau fixant comprenant au moins un polymère siliconé greffé anionique, à squelette polysiloxanique greffé par au moins un radical hydrocarboné anionique, le dispositif étant approprié pour obtenir un pouvoir mouillant supérieur ou égal à 30 mg/s, et

possédant une valve droite avec un gicleur de diamètre compris entre 0,35 et 0,60 mm.

Le moyen de distribution est donc constitué par une valve de distribution commandée par une tête de distribution, elle même comprenant une buse par laquelle la composition aérosol est vaporisée.

Les notions de « Tg » et « pouvoir mouillant » telles qu'on les entend selon la présente invention sont définies ci-dessous.

Par température de transition vitreuse (Tg), on entend selon la présente invention la Tg du matériau fixant dans l'extrait sec, l'extrait sec étant constitué par l'ensemble des matériaux non volatiles dans le jus, ou matière sèche.

Selon la présente invention, le pouvoir mouillant correspond à la quantité de produit reçue par une feuille de matière plastique disposée à une distance de 35 cm de la buse du dispositif aérosol pendant une unité de temps donnée. Le produit est alors constitué par la matière sèche, plus une partie du solvant non évaporé en cours du trajet, plus éventuellement une partie du propulseur non évaporé. Ce pouvoir mouillant est exprimé en mg/s, et est mesuré selon l'invention par la méthode suivante:

- on suspend verticalement une feuille de matière plastique de dimensions 21 cm x 23 cm à une balance de précision (1/1000), la feuille étant reliée à la balance par le bord supérieur (généralement par un crochet de la balance inséré dans une perforation disposée au centre de la largeur et à 1 cm du bord supérieur de la feuille), et maintenue verticale par l'application d'un poids centré sur le bord inférieur (généralement par une pince fixée et centrée sur le bord inférieur);
- on place une cale derrière le bord inférieur de la feuille pour maintenir la feuille verticale lors de l'impact du produit;
- on dispose verticalement le dispositif aérosol de manière que la buse de diffusion de la composition soit disposée au centre et à 35 cm de la feuille verticale, pour une vaporisation du produit perpendiculaire à la feuille;

- on vaporise la composition pendant 5 secondes;
- on mesure la quantité de produit reçue sur la feuille verticale dès la fin de la vaporisation.

Pour plus de précision, on peut employer un dispositif approprié comprenant un moyen support du dispositif aérosol, et des moyens permettant le réglage tridimensionnel de la position de la buse par rapport à la feuille verticale. Ce dispositif peut être également équipé d'un dispositif pneumatique de contrôle du spray (déclenchement et durée), de manière à contrôler avec précision la durée de la vaporisation. L'ensemble peut être contrôlé par un ordinateur.

Pour éviter les perturbations environnementales, le trajet du produit entre la buse et la feuille sera avantageusement protégé horizontalement et verticalement par les parois d'un tunnel de dimensions appropriées.

Enfin, la vaporisation du produit est avantageusement effectuée sous atmosphère contrôlée, de manière préférentielle à une température de 20°C et une humidité relative de 30%.

De manière préférentielle, le matériau fixant a une Tg supérieure ou égale à 60 °C.

De manière avantageuse, le matériau fixant est essentiellement constitué par le polymère siliconé greffé anionique, seul ou en combinaison avec des additifs cosmétiques usuels, par exemple des plastifiants, ou des agents neutralisants.

Selon la présente invention, on désigne par polymère siliconé, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en $C_1$-$C_{10}$ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné anionique, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxy-éthylène et/ou polyoxypropylène, les radicaux hydroxyls ou hydroxyalkyls, les groupements aminés substitués ou non, les groupements amides, les radicaux acyloxy ou acyloxyalkyls, les radicaux hydroxyalkylamino ou aminoalkyls, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon la présente invention, on entend par radical hydrocarboné un radical hydrocarboné non siliconé.

Les polymères siliconés anioniques utiles selon la présente invention, sont ceux qui comprennent une chaîne principale de silicone (ou polysiloxane $(\equiv Si\text{-}O\text{-})_n$) sur laquelle se trouve greffé, à l'intérieur de ladite chaîne ainsi qu'éventuellement à l'une au moins de ses extrémités, au moins un radical hydrocarboné anionique non siliconé. De manière préférentielle, le radical hydrocarboné anionique est un polymère hydrocarboné anionique. Les charges anioniques peuvent être amenées par au moins une fonction ionisable, en particulier par au moins une fonction acide carboxylique ou acide sulfonique, et leurs sels d'addition avec une base, par exemple la soude, la potasse, l'amino-2 méthyl-2 propanol-1, la monoéthanolamine, la triéthanolamine, la triisopropanolamine, l'ammoniaque, etc.

De préférence, la masse moléculaire en nombre des polymères siliconés greffés anioniques selon l'invention est suppérieure à environ 5 000, de préférence comprise entre 10 000 et 1 000 000 environ, plus préférentiellement comprise entre 10 000 et 100 000 environ.

De manière préférentielle, le rapport pondéral chaîne de silicone /radical hydrocarboné anionique est compris entre 5/95 et 40/60.

Enfin, de manière avantageuse, les polymères siliconés greffés anioniques selon l'invention sont des polymères à rigidification rapide. Par polymères à rigidification rapide, on entend selon l'invention les polymères qui conduisent à des soudures rigides en moins de 10 minutes.

Les polymères siliconés greffés anioniques utiles selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques $CH_2$=CH-, ou encore la réaction entre des groupements thio-fonctionnels -SH avec ces mêmes groupements vinyliques.

Des exemples de polymères siliconés greffés anioniques convenant à la mise en oeuvre de la présente invention, ainsi que leur mode particulier de préparation, sont notamment décrits dans les demandes de brevets EP-A-0 582 152, WO 93/23009 et WO 95/03776.

Selon un mode particulièrement préféré de réalisation de la présente invention, le polymère siliconé greffé

anionique mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélange, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels de métaux alcalins, alcalino-terreux et d'ammonium. On notera que, de même, dans le polymère siliconé greffé anionique final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, potasse, ammoniaque,...) pour l'amener sous la forme d'un sel.

Selon la présente invention, les monomères hydrophobes à insaturation éthylénique sont de préférence choisis, seuls ou en mélange, parmi les esters d'acide acrylique alcanols et/ou les esters d'acide méthacrylique d'alcanols. Les alcanols sont de préférence en $C_1$-$C_{18}$ et plus particulièrement en $C_1$-$C_{12}$. Les monomères préférentiels sont choisis dans le groupe constitué par le (méth)acrylate d'isooctyle, le (méth)acrylate d'isonyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés greffés anioniques convenant particulièrement bien à la mise en oeuvre de la présente invention est constituée par les polymères siliconés comportant dans leur structure le motif de formule (I) suivant :

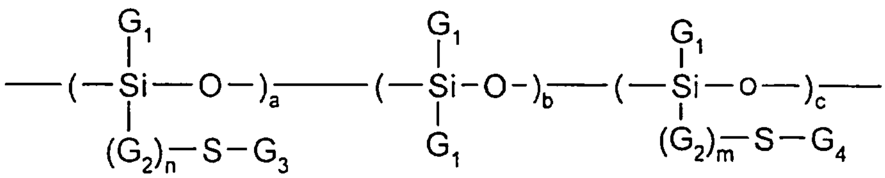

(I)

dans lequel les radicaux $G_1$, identiques ou différents, représentent l'hydrogène ou un radical alkyle en $C_1$-$C_{10}$ ou encore un radical phényle ; les radicaux $G_2$, identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de 1'(homo)polymérisation d'au moins un monomère hydrophobe à insaturation éthylénique ; m et n sont égaux à 0 ou 1 ; a est un nombre entier allant de 0 et 30 ; b est un nombre entier pouvant être compris entre 10 et 330, c est un nombre entier allant de 0 et 30 ; sous réserve que l'un des paramètres a et c soit différent de 0.

De préférence, le motif de formule (I) ci-dessus présente au moins l'une, et encore plus préférentiellement l'ensemble, des caractéristiques suivantes:

- les radicaux $G_1$ désignent un radical alkyle, de préférence le radical méthyle ;
- n est non nul, et les radicaux $G_2$ représentent un radical divalent en $C_1$-$C_3$, de préférence un radical propylène ;
- $G_3$ représente un radical polymérique résultant de l'(homo)polymérisation d'au moins un monomère du type acide carboxylique à insaturation éthylénique, de préférence l'acide acrylique et/ou l'acide méthacrylique ;
- $G_4$ représente un radical polymérique résultant de l'(homo)polymérisation d'au moins un monomère du type (méth) acrylate d'alkyle($C_1$-$C_{10}$), de préférence du type (méth)acrylate d'isobutyle ou de méthyle.

Des exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Parmi les polymères siliconés greffés anioniques utiles selon l'invention, on citera plus particulièrement le produit commercialisé sous la dénomination VS80 par la société 3 M.

Le matériau fixant peut être utilisés sous forme solubilisée ou sous forme de dispersions de particules solides de polymère.

Le dispositif aérosol selon l'invention est approprié pour obtenir un pouvoir mouillant supérieur ou égal à 30 mg/s, et de manière avantageuse, compris entre 30 mg/s et 300 mg/s.

Selon un mode préférentiel de réalisation de l'invention, le dispositif aérosol selon l'invention est approprié pour obtenir un débit en matière sèche supérieur ou égal à 20 mg/s, de préférence compris entre 20 et 60 mg/s

Selon la présente invention, le débit en matière ($D_{MS}$) sèche correspond en la quantité d'extrait sec qui sort du dispositif aérosol par unité de temps. Ce débit en matière sèche est exprimé en mg/s et est calculé en multipliant la concentration en matière sèche dans la composition aérosol ($C_{MS}$) par le débit de la composition aéroM.sol à la sortie de la buse ($D_{CA}$):

$$D_{MS} = C_{MS} \times D_{CA}.$$

La concentration en matière sèche dans la composition aérosol ($C_{MS}$) correspond à la quantité de matière sèche ramenée à 100g de composition aérosol (jus + propulseur). La concentration en matière sèche est exprimée en pourcentage et est mesurée après pulvérisation par évaporation des composants volatils du résidu de pulvérisation pendant 1 heure 30 à 105 °C.

Le débit en composition aérosol ($D_{CA}$) correspond à la quantité de composition aérosol (jus + propulseur) sortant du dispositif aérosol par unité de temps. Il est exprimé en mg/s et est mesuré par la différence entre le poids de l'aérosol avant ($M_0$) et après ($M_1$) 10 secondes de vaporisation:

$$D_{CA} = (M_0 - M_1) / 10.$$

Les caractéristiques de débit en matière sèche et de pouvoir mouillant des dispositifs aérosols selon l'invention dépendent d'une part de la composition aérosol, et d'autre part au moyen de distribution, les deux devant être appropriés pour obtenir les caractéristiques recherchées. Parmi les paramètres susceptibles d'influencer ces caractéristiques, on citera plus particulièrement la concentration en matière sèche ($C_{MS}$), le débit en composition aérosol ($D_{CA}$) et la phase de la composition aérosol.

D'une manière avantageuse, la concentration en matière sèche ($C_{MS}$) est comprise entre 2,5 et 15 % en poids par rapport au poids total de la composition aérosol (jus + propulseur), de préférence comprise entre 3,5 et 10 % en poids.

Le débit en composition aérosol ($D_{CA}$) sera alors approprié pour obtenir un débit en matière sèche ($D_{MS}$) tel que défini ci-dessus. De manière préférentielle, le $D_{CA}$ sera compris entre 300 et 800 mg/s, plus préférentiellement voisin de 600 mg/s.

La phase de la composition aérosol est de préférence une phase longue, c'est à dire que le rapport pondéral jus/propulseur est supérieur à 1, plus préférentiellement compris entre 1,2 et 3.

De manière avantageuse, le solvant approprié contient au moins 30 % en volume d'alcool, de préférence au moins 70 % en volume d'alcool. Par alcool on entend selon l'invention un alcool aliphatique en $C_1$-$C_4$, de préférence l'éthanol.

Le propulseur est constitué par les gaz comprimés ou liquéfiés usuellement employés pour la préparation de compositions aérosols. On emploiera de manière préférentielle l'air, le gaz carbonique ou l'azote comprimés, ou encore un gaz soluble ou non dans la composition tel que le diméthyl éther, les hydrocarbures fluorés ou non, et leurs mélanges.

La composition aérosol selon l'invention peut en outre comprendre d'autes polymères fixants, dans la mesure où ils ne viennent pas modifier les caractéristiques du dispositif selon l'invention, en particulier pour ce qui concerne la valeur de Tg du matériau fixant. Ces polymères fixants sont notamment les copolymères d'acide acrylique tels que le terpolymère acide acrylique /acrylate d'éthyle / N-tertiobutylacrylamide (en particulier commercialisé sous la dénomination ULTRAHOLD STRONG par la société BASF), ou encore les polymères à motifs vinyl lactames utiles selon l'invention, on citera plus particulièrement les polyvinyl pyrrolidones, les polyvinyl caprolactames, les copolymères polyvinyl pyrrolidone / acétate de vinyle, les copolymères polyvinyl pyrrolidone /méthacrylate de diaminoéthyle non quaternisé (en particulier commercialisés sous les dénominations Copolymère 845, Copolymère 958 et Copolymère 937 par la société ISP), les terpolymères polyvinyl pyrrolidone / acétate de vinyle /propionate de vinyle (en particulier commercialisés sous la dénomination Luviskol VAP 343 par la société BASF), les terpolymères vinyl caprolactame / vinyl pyrrolidone / méthacrylate de diméthyl amino éthyle (en particulier commercialisé sous les dénominations Gaffix VC 713, $H_2$OLD, ACP 1187 et ACP 1189 par la société ISP) et les terpolymères acide (méth)acrylique / (méth)acrylates / vinyl pyrrolidone (en particulier commercialisés sous les dénominations Stepanhold Extra par la société STEPAN, Luvimer VBM 35 et Luvimer

VBM 70 par la société BASF), ou encore le terpolymère acide crotonique / acétate de vinyle /t.butyl benzoate de vinyle.

En fonction de la composition aérosol (jus + propulseur), l'homme du métier saura sélectionner le moyen de distribution approprié pour obtenir les caractéristiques de débit en matière sèche et de pouvoir mouillant recherchées.

Les caractéristiques particulières définies ci-dessus ($C_{MS}$ et phase), peuvent être obtenues en sélectionnant les moyens de distribution appropriés et/ou en agissant sur la formulation.

Les valves appropriées pour les compositions selon l'invention sont des valves droites avec un gicleur de diamètre compris entre 0,35 et 0,60 mm, de préférence compris entre 0,40 et 0,30 mm, avantageusement sans restriction interne ni prise de gaz additionnelle. Il s'agit en particulier des valves commercialisées sous la dénomination COSTER T104 RA36/014 par la société COSTER, ou de la valve Précision Expérimentale 15130 constituée par un gicleur et un corps de valve de 0,46 mm de diamètres sans prise de gaz additionnelle de la société Précision.

Les diffuseurs appropriés pour les compositions particulières ci-dessus sont en particulier les boutons poussoirs commercialisés sous la dénomination Précision 216903-40AD29 par la société Précision.

La présente invention concerne également un procédé de traitement des fibres kératiniques, dans lequel on applique sur lesdites fibres une composition comprenant un matériau fixant ayant une température de transition vitreuse (Tg) supérieure ou égale à 30 °C comprenant au moins un polymère siliconé greffé anionique tel que défini ci-dessus, au moyen d'un dispositif approprié pour obtenir un pouvoir mouillant supérieur ou égal à 30 mg/s.

Les exemples ci-après permettent d'illustrer l'invention sans toutefois en limiter la portée. Dans les exemples, « ma » signifie « matière active ».

## Exemple 1

On prépare cinq jus comprenant 5,7 % en poids de polymères fixants dans l'éthanol pour les cinq polymères fixants suivants:

A- VS 80 (commercialisé par la société 3M), polymère siliconé greffé anionique selon l'invention,
B- terpolymère de méthyl siloxane à groupements méthyl thio / méthacrylate de diméthylaminoéthyle / méthacryllate d'isobutyle (20/10/70), polymère siliconé greffé cationique en dehors de la présente invention,
C- copolymère diméthyl / méthyl siloxane à groupements ester polyacryliques, polymère siliconé greffé non ionique en dehors de la présente invention,
D- terpolymère acide acrylique / acrylate de tertiobutyle / méthacrylate à groupements PDMS greffés (20/60/20), décrits dans la demande de brevet EP 412 704, en dehors de la présente invention, .
E- octylacrylamide / acrylates / butylaminoethylmethacrylate (CTFA) commercialisés sous la dénomination AMPHOMER par la société NATIONAL STARCH, en dehors de la présente invention.

On prépare cinq dispositifs aérosols 1 à 5 en introduisant dans des flacons aérosol 65 g des compositions A à E, respectivement. On équipe les cinq flacons d'une valve Précision Expérimentale 15130, puis on ajoute 35 g de diméthyl éther comme propulseur, et un bouton poussoir Précision 216903-40AD29.

Les cinq dispositifs ci-dessus sont testés sur mèches comme suit:

- on prépare une mèche de cheveux lavés et séchés d'environ 5 g,
- on étale les cheveux en éventail et on suspend la mèche,
- on pulvérise la composition 5 secondes de chaque coté,
- on attend que la mèche soit sèche (environ 10 minutes).

Les performances de chaque dispositif sont ensuites évaluées par un panel de huit experts qui notent en aveugle les paramètres suivants: pouvoir laquant, qualité du toucher, qualité visuelle et facilité de démêlage. Les performances sont notées de 0 à 30, 0 correspondant à des performances inacceptables, 30 à d'excellentes performances.

Les moyennes obtenues pour les cinq dispositifs sont reportées dans le Tableau ci-dessous

| Dispositif | 1 | 2* | 3* | 4* | 5* |
|---|---|---|---|---|---|
| Polymère | A | B | C | D | E |
| Pouvoir laquant | 35 | 15 | 15 | 35 | 35 |
| Toucher | 32 | 5 | 5 | 17 | 17 |
| Visuel | 40 | 5 | 5 | 25 | 30 |

Suite de tableau

| | | | | | |
|---|---|---|---|---|---|
| Démêlage | 40 | 35 | 40 | 20 | 12 |
| * Comparatifs | | | | | |

Seul le dispositif 1 selon l'invention permet d'obtenir une note satisfaisante pour les quatre critères retenus, les quatre autres dispositifs donnant des résultats inférieurs, tant au niveau du pouvoir laquant, du toucher ou du visuel (dispositifs 2 et 3) que de la facilité de démêlage (dispositifs 4 et 5).

## Exemple 2

On prépare le jus suivants:

| | |
|---|---|
| VS80 commercialisé par la société 3M | 3,0 g ma |
| Terpolymère acide acrylique / acrylate d'éthyle / N-tertiobutylacrylamide commercialisé sous la dénomination ULTRAHOLD STRONG par la société BASF | 3,0 g ma |
| Ethanol qs | 100,00 g |

On prépare le dispositif aérosol selon l'invention en introduisant dans un flacon aérosol 65 g du jus ci-dessus. On équipe le flacon d'une valve Précision Expérimentale 15130, puis on ajoute 35 g de diméthyl éther comme propulseur, et un bouton poussoir Précision 216903-40AD29.

Ce dispositif selon l'invention permettent d'obtenir les résultats de fixation cosmétique, de toucher et de démêlage conformes à l'invention.

## Revendications

1. Dispositif aérosol constitué par un récipient contenant une composition aérosol constituée d'une part par une phase liquide (ou jus) contenant au moins un matériau fixant dans un solvant approprié et d'autre part un propulseur, et un moyen de distribution de ladite composition aérosol, **caractérisé en ce que** le matériau fixant a une température de transition vitreuse (Tg) supérieure ou égale à 30 °C, ledit matériau fixant comprenant au moins un polymère siliconé greffé anionique à squelette polysiloxanique greffé par au moins un radical hydrocarboné anionique, et **en ce que** le dispositif est approprié pour obtenir un pouvoir mouillant supérieur ou égal à 30 mg/s, et possède une valve droite avec un gicleur de diamètre compris entre 0,35 et 0,60 mm.

2. Dispositif selon la revendication 1, **caractérisé en ce que** le polymère siliconé greffé anionique comprend une chaîne principale de silicone (ou polysiloxane $(\equiv Si\text{-}O\text{-})_n$) sur laquelle se trouve greffé, à l'intérieur de ladite chaîne ainsi qu'éventuellement à l'une au moins de ses extrémités, au moins un radical hydrocarboné anionique non siliconé.

3. Dispositif selon l'une des revendications 1 ou 2, **caractérisé en ce que** le radical hydrocarboné anionique comprend au moins une fonction acide carboxylique ou acide sulfonique, et leurs sels d'addition avec une base.

4. Dispositif selon l'une des revendications 1 à 3, **caractérisé en ce que** le rapport pondéral chaîne de silicone / groupement hydrocarboné anionique est compris entre 5/95 et 40/60.

5. Dispositif selon l'une des revendications 1 à 4, **caractérisé en ce qu'**il est approprié pour obtenir un pouvoir mouillant compris entre 30 mg/s et 300 mg/s.

6. Dispositif selon l'une des revendications 1 à 5, **caractérisé en ce qu'**il est approprié pour obtenir un débit en matière sèche supérieur ou égal à 20 mg/s, de préférence compris entre 20 et 60 mg/s.

7. Dispositif selon l'une des revendications 1 à 6, **caractérisé en ce que** la concentration en matière sèche ($C_{MS}$) est comprise entre 2,5 et 15 % en poids par rapport au poids total de la composition aérosol (jus + propulseur).

8. Dispositif selon l'une des revendications 1 à 7, **caractérisé en ce que** le débit en composition aérosol ($D_{CA}$) est compris entre 300 et 800 mg/s, de préférence voisin de 600 mg/s.

**9.** Dispositif selon l'une des revendications 1 à 8, **caractérisé en ce que** le rapport pondéral jus/propulseur est supérieur à 1, de préférence compris entre 1,2 et 3.

**10.** Dispositif selon l'une des revendications 1 à 9, **caractérisé en ce que** le solvant approprié contient au moins 30 % en volume d'alcool, de préférence au moins 70 % en volume d'alcool.

**11.** Dispositif selon l'une des revendications 1 à 10, **caractérisé en ce qu'**il comprend d'autres polymères fixants.

**12.** Procédé de traitement des fibres kératiniques, **caractérisé en ce qu'**on applique sur lesdites fibres une composition comprenant un matériau fixant ayant une température de transition vitreuse (Tg) supérieure ou égale à 50 °C, ledit matériau fixant comprenant au moins un polymère siliconé anionique tel que défini dans les revendications 1 à 6, au moyen d'un dispositif approprié pour obtenir un pouvoir mouillant supérieur ou égal à 30 mg/s, et possédant une valve droite avec un gicleur de diamètre compris entre 0,35 at 0,60mm.

## Claims

**1.** Aerosol device consisting of a container containing an aerosol composition consisting, on the one hand, of a liquid phase (or fluid) containing at least one fixing material in a suitable solvent and, on the other hand, a propellant, and a means for distributing the said aerosol composition, **characterized in that** the fixing material has a glass transition temperature (Tg) of greater than or equal to 30°C, the said fixing material comprising at least one anionic grafted silicone polymer, having a polysiloxane skeleton grafted with at least one anionic hydrocarbon radical, and **in that** the device is suitable for obtaining a wetting power of greater than or equal to 30 mg/s, and has a straight-line valve with a spray nozzle having a diameter of between 0.35 and 0.60 mm.

**2.** Device according to Claim 1, **characterized in that** the anionic grafted silicone polymer comprises a main silicone chain (or polysiloxane $(\equiv \text{Si-O-})_n$) onto which at least one non-silicone anionic hydrocarbon radical is grafted, within the said chain as well as, optionally, on at least one of its ends.

**3.** Device according to either of Claims 1 and 2, **characterized in that** the anionic hydrocarbon radical comprises at least one carboxylic acid or sulphonic acid function, and their addition salts with a base.

**4.** Device according to one of Claims 1 to 3, **characterized in that** the silicone chain/anionic hydrocarbon group weight ratio is between 5/95 and 40/60.

**5.** Device according to one of Claims 1 to 4, **characterized in that** it is suitable for obtaining a wetting power of between 30 mg/s and 300 mg/s.

**6.** Device according to one of Claims 1 to 5, **characterized in that** it is suitable for obtaining a solids flow rate of greater than or equal to 20 mg/s, preferably between 20 and 60 mg/s.

**7.** Device according to one of Claims 1 to 6, **characterized in that** the solids concentration ($C_{SM}$) is between 2.5 and 15% by weight relative to the total weight of the aerosol composition (fluid + propellant).

**8.** Device according to one of Claims 1 to 7, **characterized in that** the flow rate of aerosol composition ($D_{AC}$) is between 300 and 800 mg/s, preferably close to 600 mg/s.

**9.** Device according to one of Claims 1 to 8, **characterized in that** the fluid/propellant weight ratio is greater than 1, preferably between 1.2 and 3.

**10.** Device according to one of Claims 1 to 9, **characterized in that** the appropriate solvent contains at least 30% by volume of alcohol, preferably at least 70% by volume of alcohol.

**11.** Device according to one of Claims 1 to 10, **characterized in that** it comprises other fixing polymers.

**12.** Process for treating keratin fibres, **characterized in that** a composition comprising a fixing material having a glass transition temperature (Tg) of greater than or equal to 50°C, this fixing material comprising at least one anionic silicone polymer as defined in Claims 1 to 6, is applied to the said fibres by means of a device which is suitable for

obtaining a wetting power of greater than or equal to 30 mg/s, and having a straight-line valve with a spray nozzle having a diameter of between 0.35 and 0.60 mm.

## Patentansprüche

1. Aerosolvorrichtung, die aus einem Behälter, der eine Aerosolzusammensetzung enthält, die zum einen aus einer flüssigen Phase (oder Flüssigkeit), die mindestens ein festigendes Material in einem geeigneten Lösemittel enthält, und zum anderen aus einem Treibmittel besteht, und einer Einrichtung zum Verteilen der Aerosolzusammensetzung besteht, **dadurch gekennzeichnet, dass** das festigende Material eine Glasübergangstemperatur (Tg) von 30 °C oder darüber aufweist, wobei das festigende Material mindestens ein anionisches gepfropftes Siliconpolymer mit Polysiloxangerüst umfasst, auf das mindestens ein anionischer Kohlenwasserstoffrest gepfropft ist, und dass die Vorrichtung dafür geeignet ist, ein Benetzungsvermögen von 30 mg/s oder darüber zu erhalten und dass sie ein gerades Ventil mit einer Spritzdüse mit einem Durchmesser besitzt, der im Bereich von 0,35 bis 0,60 mm liegt.

2. Vorrichtung nach Anspruch 1, **dadurch gekennzeichnet, dass** das anionische gepfropfte Siliconpolymer eine Siliconhauptkette (oder Polysiloxan $(\equiv Si\text{-}O)_n$) umfasst, auf die im Inneren der Kette und gegebenenfalls an mindestens einem ihrer Enden mindestens ein nicht siliconhaltiger anionischer Kohlenwasserstoffrest gepfropft ist.

3. Vorrichtung nach Anspruch 1 oder 2, **dadurch gekennzeichnet, dass** der anionische Kohlenwasserstoffrest mindestens eine Carbonsäure- oder Sulfonsäuregruppe, und ihre Additionssalze mit einer Base umfasst.

4. Vorrichtung nach einem der Ansprüche 1 bis 3, **dadurch gekennzeichnet, dass** das Gewichtsverhältnis Siliconkette/anionische Kohlenwasserstoffgruppe im Bereich von 5/95 bis 40/60 liegt.

5. Vorrichtung nach einem der Ansprüche 1 bis 4, **dadurch gekennzeichnet, dass** sie dafür geeignet ist, ein Benetzungsvermögen zu erhalten, das im Bereich von 30 bis 300 mg/ s liegt.

6. Vorrichtung nach einem der Ansprüche 1 bis 5, **dadurch gekennzeichnet, dass** sie dafür geeignet ist, einen Durchsatz an Trockensubstanz von 20 mg/ s oder darüber zu erhalten, der vorzugsweise im Bereich von 20 bis 60 mg/s liegt.

7. Vorrichtung nach einem der Ansprüche 1 bis 6, **dadurch gekennzeichnet, dass** die Konzentration an Trockensubstanz ($C_{TS}$) im Bereich von 2,5 bis 15 Gew.-%, bezogen auf das Gesamtgewicht der Aerosolzusammensetzung (Flüssigkeit + Treibmittel), liegt.

8. Vorrichtung nach einem der Ansprüche 1 bis 7, **dadurch gekennzeichnet, dass** der Durchsatz an Aerosolzusammensetzung ($D_{AZ}$) im Bereich von 300 bis 800 mg/ s und vorzugsweise bei etwa 600 mg/ s liegt.

9. Vorrichtung nach einem der Ansprüche 1 bis 8, **dadurch gekennzeichnet, dass** das Gewichtsverhältnis Flüssigkeit/Treibmittel größer als 1 ist und vorzugsweise im Bereich von 1,2 bis 3 liegt.

10. Vorrichtung nach einem der Ansprüche 1 bis 9, **dadurch gekennzeichnet, dass** das geeignete Lösemittel mindestens 30 Vol.-% Alkohol, vorzugsweise mindestens 70 Vol.-% Alkohol, enthält.

11. Vorrichtung nach einem der Ansprüche 1 bis 10, **dadurch gekennzeichnet, dass** sie weitere festigende Polymere enthält.

12. Verfahren zur Behandlung von Keratinfasern, **dadurch gekennzeichnet, dass** eine Zusammensetzung, die ein festigendes Material enthält, das eine Glasübergangstemperatur (Tg) von 50 °C oder darüber aufweist, wobei das festigende Material mindestens ein anionisches Siliconpolymer umfasst, das wie in den Ansprüchen 1 bis 6 definiert ist, mit Hilfe einer Vorrichtung aufgetragen wird, die dafür geeignet ist, ein Benetzungsvermögen von 30 mg/ s oder darüber zu erhalten und die ein gerades Ventil mit einer Spritzdüse mit einem Durchmesser, der im Bereich von 0,35 bis 0,60 mm liegt, besitzt.